(19) Europäisches Patentamt
European Patent Office
Office européen des brevets

(11) Publication number: 0 375 393 A1

# (12) EUROPEAN PATENT APPLICATION

(21) Application number: 89313349.6

(22) Date of filing: 20.12.89

(51) Int. Cl.5: C07H 17/08, C07D 493/22, A61K 31/70, A01N 43/90

(30) Priority: 23.12.88 US 289118

(43) Date of publication of application: 27.06.90 Bulletin 90/26

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Mrozik, Helmut
159 Idlebrook Lane
Matawan New Jersey 07747(US)

(74) Representative: Hesketh, Alan, Dr. et al
European Patent Department Merck & Co., Inc. Terlings Park Eastwick Road
Harlow Essex, CM20 2QR(GB)

## (54) Avermectin derivatives.

(57) Synthetically modified avermectin derivatives are disclosed containing novel alkyl, and alkenyl substituents at position 25 and having additional substituents at positions 4′- and 4″- of the oleandrose structural part. The compounds are prepared from the known C-25 substituted avermectin derivatives by the appropriate chemical reactions at the 4′-, and 4″-positions. The new compounds are potent antiparasitic agents, in particular, the compounds are anthelmintic, insecticidal and acaricidal agents.

EP 0 375 393 A1

# AVERMECTIN DERIVATIVES

## BACKGROUND OF THE INVENTION

Certain avermectin derivatives with novel C-25 substituents other than the natural 2-propyl or 2-butyl groups are known in the art, and are described in the European Patent Application EPO 0214 731. The instant compounds are derivatives of such compounds which contain new or modified substituents at the 4′-, and 4″-positions.

The term avermectin (previously referred to as C-076) is used to describe a series of compounds isolated from the fermentation broth of an avermectin producing strain of Streptomyces avermitilis and derivatives thereof. The morphological character istics of the culture are completely described in U.S. Pat. No. 4,310,519 and are incorporated herein by reference. The avermectin compounds are a series of macrolides, each of which is substituted thereon at the 13-position with a 4′-(a-L-oleandrosyl)-a-L-oleandrose group. Avermectin compounds and the derivatives thereof of this invention have a very high degree of anthelmintic and antiparasitic activity.

## SUMMARY OF THE INVENTION

The instant invention is concerned with derivatives of avermectins where microbially produced avermectin compounds with novel alkyl and alkenyl substituents at position 25 with preexisting hydroxy group substituents at positions 4′- and 4″- are converted by synthetic chemical reactions to derivatives with other substituents at such positions. Thus it is an object of this invention to describe such compounds. It is a further object of this invention to describe the processes useful for the preparation of such compounds. A still further object is to describe the use of such compounds as anthelmintic, insecticidal and aoaricidal agents. Still further objects will become obvious from a reading of the following description.

## DESCRIPTION OF THE INVENTION

The compounds of the instant invention are best realized in the following structural formula:

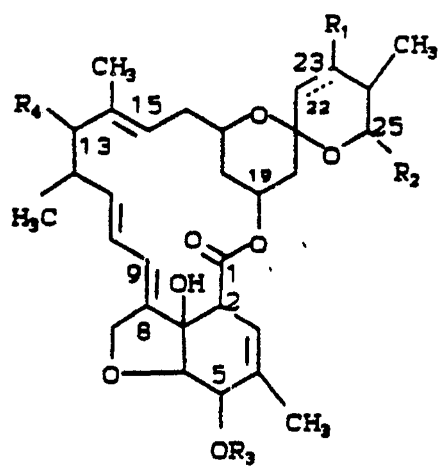

wherein the broken line at the 22,23-position represents a single bond and wherein $R_1$ is hydrogen or hydroxy, or the broken line represents a double bond and $R_1$ is absent;

$R_2$ is an alpha-branched $C_3$-$C_8$ alkyl, alkenyl, alkoyalkyl or alkylthioalkyl group; a $C_5$-$C_8$ cycloalkyl substituted $C_1$-$C_4$ alkyl group; a $C_3$-$C_8$ cycloalkyl or $C_5$-$C_8$ cycloalkenyl group, either of which may optionally be substituted by methylene or one or more $C_1$-$C_4$ alkyl groups or halo atoms; or a 3 to 6 membered oxygen or sulfur containing heterocyclic ring which may be saturated, or fully or partly unsaturated and which may optionally be substituted by one or more $C_1$-$C_4$ alkyl groups or halo atoms, provided that $R_2$ is not 2-propyl, 2-butyl, 2-buten-2-yl, 2-penten-2-yl or 4-methyl-2-penten-2-yl;

$R_3$ is hydrogen, loweralkyl, or loweralkanoyl;
$R_4$ is

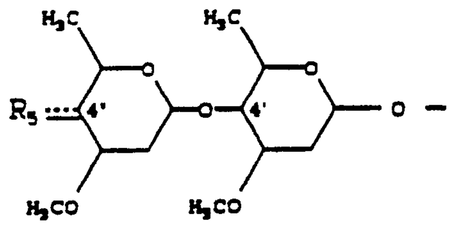
or
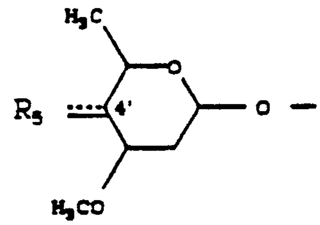

where the broken line at the $4'$ and $4''$ positions indicates that $R_5$ is connected to the saccharide moiety through a double bond when $R_5$ is ketone, oxime, hydrazone, loweralkanoylhydrazone, or semicarbazone, N-loweralkylsemicarbazone, N,N-diloweralkylsemicarbazone, or $R_5$ is connected to the saccharide moiety through a single bond when $R_5$ is amino, loweralkylamino, diloweralkylamino, lower alkanoylamino, loweralkoxycarbonylamino, carbamoyloxy, N-loweralkylcarbamoyloxy, N,N-diloweralkylcarbamoyloxy, loweralkoxy, loweralkanoyloxy, or loweralkoxycarbonyloxy.

Preferred compounds of the instant invention are realized in the forgoing structural formula wherein the broken line at the 22,23-position represents a single bond and wherein $R_1$ is hydrogen or hydroxy, or the broken line represents a double bond and $R_1$ is absent;
$R_2$ is an alpha branched $C_3$-$C_8$ alkyl or alkenyl group, a $C_5$-$C_8$ cycloalkyl substituted $C_1$-$C_4$ alkyl group; a $C_3$-$C_8$ cycloalkyl or $C_5$-$C_8$ cycloalkenyl group, either of which may optionally be substituted by methylene or one or more $C_1$-$C_4$ alkyl groups or halo atoms;
$R_3$ is hydrogen;
$R_4$ is

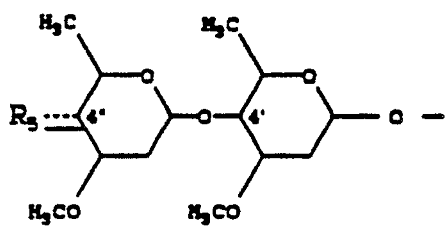
or
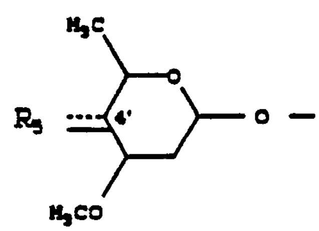

where the broken line at the $4'$ and $4''$ positions indicates that $R^5$ is connected by a double bond and $R_5$ is ketone, loweralkanoylhydrazone, semicarbazone or N-loweralkylsemicarbazone or N,N-diloweralkylsemicarbazone, or the broken line at the $4'$ and $4''$ positions indicates $R^5$ is connected by a single bond and $R_5$ is amino, loweralkylamino, diloweralkylamino, lower alkanoylamino, loweralkoxycarbonylamino, carbamoyloxy, N-loweralkyl carbamoyloxy, or N,N-diloweralkylcarbamoyloxy.

The most preferred compounds are realized wherein the broken line at the 22,23-position represents a single bond and wherein $R_1$ is hydrogen or hydroxy, or the broken line represents a double bond and $R_1$ is absent;
$R_2$ is 2-pentyl, 2-hexyl, cyclobutyl, cyclopentyl, cyclohexyl, 3-thiophenyl, 1-methylthioethyl;
$R_3$ is hydrogen;
$R_4$ is

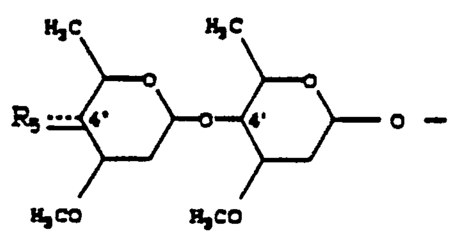

where the broken line at the 4″-position indicates that $R^5$ is connected by a double bond and $R_5$ is ketone, loweralkanoylhydrazone, semicarbazone, N-loweralkylsemicarbazone or N,N-diloweralkylsemicarbazone, or the broken line at the 4″ position indicates that $R^5$ is connected by a single bond and $R_5$ is amino, loweralkylamino, diloweralkylamino, lower alkanoylamino or loweralkyloxycarbonylamino.

Preferred Compounds of the instant invention are further realized in the following compounds:

25-Cyclopentyl-25-de-(1-methylpropyl )-4″-oxoavermectin A2a

4″-Amino-25-Cyclopentyl-25-de-(1-methylpropyl)-4″-deoxyavermectin A2a

4″-Acetylamino-25-cyclopentyl-25-de-(1-methylpropyl)4″-deoxyavermectin A2a

25-Cyclobutyl-25-de-(1-methylpropyl)-4″-deoxy-4″-epimethylaminoavermectin B1a

25-Cyclobutyl-25-de-(1-methylpropyl)-4″-deoxy-22,23-dihydro-4″-methylaminoavermectin B1a

4″-Acetylamino-25-de-(1-methylpropyl)-4″-deoxy-25-(1- methylpentyl)avermectin $B_2$a

4″-Acetylamino-25-de-(1-methylpropyl)-4″,23-dideoxy-25-(1-methylpentyl)avermectin B2a

25-De-(1-methylpropyl)-25-(1-methylpentyl)-4″-oxoavermectin B2a semicarbazone

25-De-(1-methylpropyl)-4″-deoxy-25-(1-methylbutyl)-22,23-dihydro-4″-epi-methylaminoavermectin B1a

25-Cyclohexyl-25-de-(1-methylpropyl)-4″-deoxy-22,23-dihydroavermectin B1a 4″-acetylhydrazone

25-De-(1-methylpropyl)-4″-deoxy-22,23-dihydro-25-(3-thiophenyl)avermectin B1a 4″-(N4,N4-dimethylsemicarbazone)

25-De-(1-methylpropyl)-4″-deoxy-22,23-dihydro-4″-methoxycarbonylamino-25-(1-methylthioethyl)avermectin B1a.

In the instant invention the term "loweralkyl" is intended to indicate those alkyl groups of from 1 to 6 carbon atoms such as methyl, ethyl, propyl, isopropyl, butyl, pentyl, hexyl and the like.

The term "loweralkoxy" is intended to include those alkoxy groups of from 1 to 6 carbon atoms such as methoxy, ethoxy, propoxy, isopropoxy, butoxy, pentoxy, hexoxy and the like.

The term "loweralkanoyl" is intended to include thos alkanoyl groups of from 2 to 6 carbon atoms such as acetyl, propionyl, butyryl, pentanoyl, hexanoyl and the like.

The term "carbamoyl" is intended to include the amino carbonyl group ($H_2NCO$-).

The term "halogen" is intended to include the halogen atoms, fluorine, chlorine, bromine or iodine.

The above structural formula is shown without a definitive stereochemistry. However, during the course of the synthetic procedures used to prepare such compounds, the products of such procedures can be a mixture of stereoisomers. In particular, the stereoisomers at the 13- and 23-positions may be oriented either a- or β-representing such groups being below or above the general plane of the molecule respectively. In each such case both the a-and β-configurations are intended to be included within the ambit of this invention.

## Preparation of Starting Materials:

The avermectins are products of microbial fermentations of the actinomycete Streptomycin avermitilis. The microbes use acetates and propionates as building blocks for most of their carbon chain, which is then further modified by enzymes of the microbes to give the completed avermectin molecules. It is known, however, that the carbon C-25 and the 2-propyl and 2-butyl substituents at this carbon are not derived from acetate or propionate units, but are derived from aminoacids L-valine and L-isoleucine, respectively. While we do not wish to be bound by theory, it is proposed, that these aminoacids are deaminated to the corresponding 2-ketoacids, and that these then are decarboxylated to give 2-methylbutyric and 2-methylpropionic acids. These acids are then directly incorporated into the avermectin structures to give the 2-propyl and 2-butyl C-25 substituents. It was also found that additions of large amounts of other acids such as cyclopentanoic, cyclobutyric, 2-methylpentanoic, 2-methylhexanoic, thiophene-3-carboxylic acids to the fermentation broth of S. avermitilis causes the microbes to accept these acids as substitutents and to make small amounts of avermectins containing these acids in the form of new C-25 substituents. It was now found that modifications of these new avermectin derivatives at the 4″-position, especially introduction of amino containing groups, increases the anthelmintic and pesticidal activities of such compounds.

The starting materials for the instant compounds are among those disclosed in European Patent Application No. 0 214 731 and have the following structures:

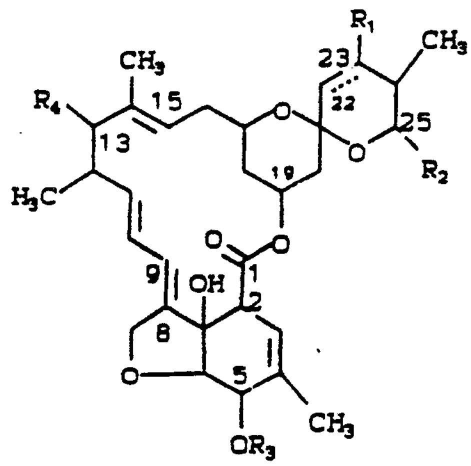

where $R_1$ and $R_2$ are as defined above;
where $R_3$ is hydrogen or methyl;
and where $R_4$ is

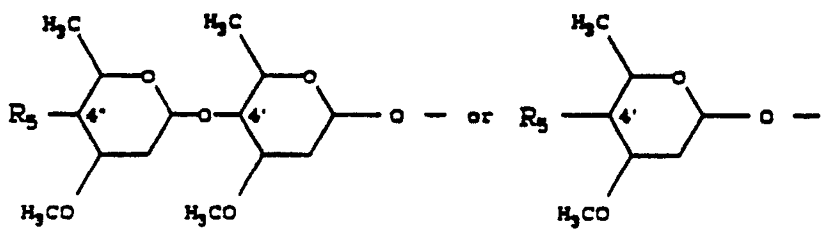

and $R_5$ is hydroxy.

Preparation of Compounds

The reactions used to convert the above compounds to the compounds of the instant invention vary depending upon the particular position of the reaction or upon the particular substituent group being prepared.

It is often useful to protect reactive hydroxy groups in order to ensure that the reaction takes place only at the desired position. Subsequent to the desired reaction or reactions, the protective group is removed. A very useful protective group is a trisubstituted silyl group, particularly the trialkyl silyl, most particularly the t-butyl-dimethylsilyl group. The protection is generally carried out at the 5-hydroxy group by combining the unprotected compound in an aprotic solvent such as methylene chloride, toluene, benzene, ethyl acetate, tetrahydrofuran, dimethylformamide and the like and adding the protecting reagent which is the silyl halide of the protecting group. The preferred reagent is tert-butyl-dimethylsilyl chloride. Also, in order to minimize side reactions, there is included in the reaction mixture a base to react with the acid halide released during the course of the reaction. Preferred amines are imidazole, pyridine or triethylamine. The base is required in amounts equimolar to the amount of hydrogen halide liberated; however, generally several equivalents of the amine are employed. The reaction is stirred at from 0° C to the reflux temperature of the reaction mixture and is complete in from 1/2 to 16 hours.

The protecting groups are removed by treatment with p-toluene sulfonic acid monohydrate in methanol at about room temperature for up to 2 hours or with a mixture of HF-pyridine in THF at room temperature for 6 to 48 hours.

Other protecting groups, such as various acyl groups are readily employed in the preparation and removal of such groups is well within the knowledge of those skilled in the art.

The monosaccharide and disaccharide compounds with hydroxy at the 4′ or 4″ positions may be converted to the 4′ or 4″ keto compounds and then to the 4′ or 4″ amino compounds.

In the first step, the avermectin starting materials are oxidized at the 4′ or 4″-position to the

corresponding keto compound. During the procedure the presence of a hydroxy group at the 5-position will require that such hydroxy group be protected in order that it too is not oxidized. The 23-hydroxy group is less reactive and the 7-hydroxy group is very unreactive and inert and they need not be protected. The procedure used to prepare the protected intermediates are described above. The oxidation reaction is carried out in an inert solvent such as methylene chloride using oxalyl chloride or trifluoroacetic anhydride and dimethylsulfoxide as the oxidizing agent. Additionally, N-chlorosuccinimide and dimethylsulfide may be employed. The reaction proceeds by dissolving the oxalyl chloride or trifluoroacetic anhydride and dimethylsulfoxide (or other oxidizing reagents) in methylene chloride with cooling from -50° to -80°C and adding dropwise a methylene chloride solution of the avermectin compound to be oxidized. The addition is carried out over a period of from 15 minutes to 1 hour and then triethylamine is added dropwise over a period of from 1 to 15 minutes. The reaction mixture is then allowed to warm to room temperature over a period of from 1/2 to 1 hour. The 4′ or 4″-keto compound is isolated using techniques known to those skilled in the art.

In the next step, the 4′ or 4″-keto compound is aminated to prepare the unsubstituted amino compound. The reaction is carried out in an inert solvent such as methanol at from -25° to +10°C using ammonium salts and sodium cyanoborohydride as the aminating and reducing reagents. The reaction is complete in from 15 minutes to 2 hours and the product 4″-deoxy-4″-amino compound (or the corresponding 4′ compound) is isolated using techniques known to those skilled in the art. Suitable ammonium salts are the acetate, propionate, benzoate and the like. The acetate is preferred.

As a variation to the foregoing amination reaction, alkyl ammonium salts could be used in place of the ammonium salts to prepare the mono alkyl substituted compounds directly. The same reagents, salts and reaction conditions as described above can be used for such a reaction.

The substitution reaction of the newly formed amino group wherein the substituent is an acyl function is carried out using an acylating reagent in the presence of a base in an inert solvent. The preferred acylating reagents are loweralkanoyl anhydrides, loweralkanoyl halides, substituted benzene sulfonyl chlorides, lower alkyl sulfonyl chlorides, and the like. The reaction is carried out in an inert solvent such as methylene chloride in the presence of a non-reactive base such as pyridine or triethylamine in order to neutralize the acid produced during the course of the reaction. The reaction temperature is from -10° to 25°C and the reaction is complete in from 5 minutes to 1 hour. The product is isolated using known techniques.

The reaction for the preparation of the 4″-deoxy-4″-dimethylamino compounds (or the corresponding 4′-compound) is carried out using the alkylating reaction conditions of formaldehyde and a reducing agent such as sodium borohydride, in methanol. The reaction is carried out in aqueous medium using excess aqueous formaldehyde along with the presence of a small amount of acid such as acetic acid to facilitate the reaction. The reaction is carried out at from -10° to +10°C with the solution of the 4″-deoxy-4″-amino-avermectin compound in methanol added dropwise over a period of from 30 to 60 minutes to the alkylating reagent mixture and the product is isolated using known techniques. See U.S. Patent 4,427,663.

The reactions which produce the various 4″ or 4′ derivatives, such as the keto, amino and substituted amino by the selective oxidation of the 4″ or 4′ hydroxy to 4″ or 4′ keto which is then reacted with an amino to prepare the amino compounds, are also detailed in U.S. 4,427,663 to Mrozik.

The 4″ or 4′ imino compounds are also prepared from the 4″ or 4′ keto compounds by reaction with the appropriately substituted semicarbazide, acyl or sulfonyl hydrazine and the like. The reaction is carried out in a non-reactive solvent at from 20 to 80°C in from 30 minutes to 48 hours. The products are isolated using techniques known to those skilled in the art.

Further reactions of avermectins are described in the chemical literature and are reviewed by Fisher M.H., Mrozik H. (1984) The avermectin family of macrolide-like antibiotics. In Omura S. (ed) Macrolide Antibiotics, Academic Press, New York, pp 553-606, and by Davies H.G., Green A.S. (1986) Avermectins and Milbemycins. Nat. Prod. Rep. 3:87-121.

The instant compounds with an acyl function at the 5, 4′ or 4″, positions are prepared by acylating the various hydroxy groups at such positions of the avermectin compounds. In particular the 5-hydroxy, 4′-hydroxy or the 4″-hydroxy, groups can be reacted with acylating agents to prepare the appropriate acylated derivative. Such compounds are prepared using the appropriate reactive intermediates such as the acid chloride, anhydride, carbamoylchloride and the like. The reaction conditions for such reactions are generally well known to those skilled in the art and are further disclosed in U.S. Patent 4,201,861 to Mrozik et. al.

The conversion of the 22,23-double bond into the reduced 22,23-single bond compound by the selective oxidation with Wilkinson's homogeneous catalyst, triphenylphosphine rhodium chloride is described in U.S. 4,199,569 to Chabala et. al.

The novel compounds of this invention have significant parasiticidal activity as anthelmintics, ectoparasiticides, insecticides and acaricides, in human and animal health and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris and Parascaris. Certain of these, such as Nematodirus, Cooperia, and Oesphagostomum attack primarily the intestinal tract while others, such as Haemonchus and Ostertagia, are more prevalent in the stomach while still others such as Dictyocaulus are found in the lungs. Still other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs and, if left untreated, may result in death of the infected host. The hydrogenated avermectin compounds of this invention have unexpectedly high activity against these parasites, and in addition are also active against Dirofilaria in dogs, Nematospiroides, Syphacia, Aspiculuris in rodents, anthropod ectoparasites of animals and birds such as ticks, mites, lice, fleas, blowfly, in sheep Lucilia sp., biting insects and such migrating diperous larvae as Hypoderma sp. cattle, Gastrophilus in horses, and Cuterebra sp. in rodents.

The instant compounds are also useful against parasites which infect humans. The most common genera of parasites of the gastro-intestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunuculus and extra intestinal stages of the intestinal worms Strongyloides and Trichinella. The compounds are also of value against arthropods parasitizing man, biting insects and other dipterous pests causing annoyance to man.

The compounds are also active against household pests such as the cockroach, Blatella sp., clothes moth, Tineola sp., carpet beetle, Attagenus sp., and the housefly Musca domestica.

The compounds are also useful against insect pests of stored grains such as Tribolium sp., Tenebrio sp. and of agricultural plants such as spider mites, (Tetranychus sp.),aphids, (Acyrthiosiphon sp.); against migratory orthopterans such as locusts and immature stages of insects living on plant tissue. The compounds are useful as a nematocide for the control of soil nematodes and plant parasites such as Meloidogyne spp. which may be of importance in agriculture.

These compounds may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Gradually, the drenches also contain an antifoaming agent. Drench formulations generally contain from about 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or di-calcium phosphate.

Where it is desired to administer the avermectin derivatives in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents, and/or binders such as starch, lactose, talc, magnesium stearate, vegetable gums and the like. Such unit dosage formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the antiparastic compounds of our invention may be administered to animals parenterally, for example, by intraruminal, intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil, cotton seed oil and the like. Other parenteral vehicles such as organic preparation using solketal, glycerol formal, and aqueous parenteral formulations are also used. The active monosaccharide or aglycone avermectin compound or compounds are dissolved or suspended in the parenteral formulation for administration; such formulations generally contain from 0.005 to 5% by weight of

the active compound.

Although the antiparasitic agents of this invention find their primary use in the treatment and/or prevention of helminthiasis, they are also useful in the prevention and treatment of diseases caused by other parasites, for example, arthropod parasites such as ticks, lice, fleas, mites and other biting insects in domesticated animals and poultry. They are also effective in treatment of parasitic diseases that occur in other animals including humans. The optimum amount to be employed for best results will, of course, depend upon the particular compound employed, the species of animal to be treated and the type and severity of parasitic infection or infestation. Generally good results are obtained with our novel compounds by the oral administration of from about 0.001 to 10 mg per kg of animal body weight, such total dose being given at one time or in divided doses over a relatively short period of time such as 1-5 days. With the preferred compounds of the invention, excellent control of such parasites is obtained in animals by administering from about 0.025 to 0.5 mg per kg of body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein are administered as a component of the feed of the animals, or dissolved or suspended in the drinking water, compositions are provided in which the active compound or compounds are intimately dispersed in an inert carrier or diluent. By inert carrier is meant one that will not react with the antiparasitic agentand one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like. The active avermectin compounds are intimately dispersed throughout the carrier by methods such as grinding, stirring, milling or tumbling. Compositions containing from about 0.005 to 2.0% weight of the active compound are particularly suitable as feed premixes. Feed supplements, which are fed directly to the animal, contain from about-0.0002 to 0.3% by weight of the active compounds.

Such supplements are added to the animal feed in an amount to give the finished feed the concentration of active compound desired for the treatment and control of parasitic diseases. Although the desired concentration of active compound will vary depending upon the factors previously mentioned as well as upon the particular avermectin derivative employed, the compounds of this invention are usually fed at concentrations of between 0.00001 to 0.00% in the feed in order to achieve the desired antiparasitic result.

In using the compounds of this invention, the individual avermectin components may be prepared and used in that form. Alternatively, mixtures of two or more of the individual avermectin components may be used, or other active compounds not related to the compounds of this invention.

The compounds of this invention are also useful in combatting agricultural pests that inflict damage upon crops while they are growing or while in storage. The compounds are applied using known techniques as sprays, dusts, emulsions and the like, to the growing or stored crops to effect protection from such agricultural pests.

The following examples are provided in order that this invention might be more fully understood; they are not to be construed as limitative of the invention.

The avermectin derivatives prepared in the following examples are generally isolated as amorphous solids and not as crystalline solids. They are thus characterized analytically using techniques such as mass spectrometry, nuclear magnetic resonance, and the like. Being amorphous, the compounds are not characterized by sharp melting points, however, the chromatographic and analytical methods employed indicate that the compounds are pure.

## EXAMPLE 1

### 25-Cyclopentyl-25-de-(1-methylpropyl)-4″-oxoavermectin A2a

To a solution containing 0.0195 ml of oxalyl chloride in 1.2 ml of dry $CH_2Cl_2$ stirred at -78°C is added

0.0319 ml of dry dimethylsulfoxide dissolved in 0.6 ml of dry $CH_2Cl_2$ during 5 min. Then a solution of 183 mg of 25-cyclopentyl-25-de-(1-methylpropyl)avermectin A2a dissolved in 1.5 ml of dry $CH_2Cl_2$ is added over a period of 10 minutes while maintaining the temperature at -78° C. The reaction mixture is stirred at this temperature for 45 minutes when 0.15 ml of dry triethylamine is added. The mixture is stirred for 5 additional minutes at -78° C, and then the cooling bath is removed and the reaction mixture is allowed to come to ambient temperature. After addition of water the reaction product is extraxted with methylene chloride, the extract is washed with water, dried and concentrated in vacuo to a yellow foam. Silicagel thin layer chromatography shows this as a mixture of three products, which are isolated by preparative silica gel layer chromatography using a 75:25 methylene chloride:ethylacetate solvent mixture. The products are identified by mass and NMR spectra as 25-cyclopentyl-25-de-(1-methylpropyl)-4″, 23-dioxo-avermectin A2a, 25-cyclopentyl-25-de-(1-methylpropyl)-4″-oxoavermectin A2a, and 25-cyclopentyl-25-de-(1-methylpropyl)-23-oxoavermectin A2a.

## EXAMPLE 1a.

When instead of 25-cyclopentyl-25-de-(1-methylpropyl)-avermectin A2 the compounds 1a) through 5a) of the following table are used as starting materials and reacted according to the procedure described in detail in example 1, the new compounds 1b) through 5b) respectively are obtained.

TABLE OF STARTING MATERIALS

1a) 25-(thien3-yl)-25-de-(1-methylpropyl) avermectin A2a
2a) 25-(cyclohex-3-enyl)-25-de-(1-methylpropyl) avermectin A2a
3a) 25-cyclohexyl-25-de-(1-methylpropyl)avermectin A2a
4a) 25-(1-methylthioethyl)-25-de-(1-methylpropyl) avermectin A2a
5a) 25-(2-methylcyclopropyl)-25-de-(1-methylpropyl) avermectin A2a

TABLE OF REACTION PRODUCTS

1b) 25-(thien-3-yl)-25-de(1-methylpropyl)-4″-oxoavermectin A2a
2b) 25-(cyclohex-3-enyl)-25-de-(1-methylpropyl)-4″-oxoavermectin A2a
3b) 25-cyclohexyl-25-de-(1-methylpropyl)-4"-oxoavermectin A2a
4b) 25-(1-methylthioethyl)-25-de-(1-methylpropyl)-4-″ oxoavermectin A2a
5b) 25-(2-methylcyclopropyl)-25-de-(1-methylpropyl)-4″ oxoavermectin A2a

## EXAMPLE 2

### 4″-Amino-25-Cyclopentyl-25-de-(1-methylpropyl)-4″-deoxyavermectin A2a

To a solution of 175 mg of 25-cyclopentyl-25-de-(1-methylpropyl)-4″-oxoavermectin A2a and 179 mg of ammonium acetate in 2.55 ml of methanol stirred at room temperature is added a solution of 19 mg of sodium cyanoborohydride in 0.5 ml of MeOH. After 30 minutes the reaction mixture is added to 30 ml of dilute aqueous sodium bicarbonate solution, and the product is extracted with ethyl acetate. The extract is washed, dried with magnesium sulfate and concentrated in vacuo to a light glass. Purification via preparative silica gel layer chromatography gives the two 4″-epimers of 4″-amino-25-cyclopentyl-25-de-(1-methylpropyl)-4″-deoxyavermectin A2a, which are isolated by preparative silica gel layer chromatography and characterized by their mass and NMR spectra.

## EXAMPLE 2a.

When instead of 25-cyclopentyl-25-de-(1-methylpropyl)-4″-oxoavermectin A2a the compounds 1b)

through 5b) of the following table are used as starting materials and reacted according to the procedure described in detail in example 2, the new compounds 1c) through 5c) respectively are obtained.

TABLE OF STARTING MATERIALS

1b) 25-(thien-3-yl)-25-de-(1-methylpropyl)-4″-oxoavermectin A2a
2b) 25-(cyclohex-3-enyl)-25-de-(1-methylpropyl)-4″-oxoavermectin A2a
3b) 25-cyclohexyl-25-de-(1-methylpropyl)-4″-oxoavermectin A2a
4b) 25-(1-methylthioethyl)-25-de-(1-methylpropyl)-4″-oxoavermectin A2a
5b) 25-(2-methylcyclopropyl)-25-de-(1-methylpropyl)-4″ oxoavermectin A2a

TABLE OF REACTION PRODUCTS

1c) 4″-amino-25-(thien-3-yl)-25-de-(1-methylpropyl)-4″-deoxyavermectin A2a
2c) 4″-amino-25-(cyclohex-3-enyl)-25-de-(1-methylpropyl)-4″-deoxyavermectin A2a
3c) 4″-amino-25-cyclohexyl-25-de-(1-methylpropyl)-4″-deoxyavermectin A2a
4c) 4″-amino-25-(1-methylthioethyl)-25-de-(1-methylpropyl)-4″-deoxyavermectin A2a
5c) 4″-amino-25-(2-methylcyclopropyl)-25-de-(1-methyl-propyl)-4″-deoxyavermectin A2a

## EXAMPLE 3

### 4″-Acetylamino-25-cyclopentyl-25-de-(1-methylpropyl)-4″-deoxyavermectin A2a

A solution of 50 mg of 4″-amino-25-cyclopentyl-25-de-(1-methylpropyl )-4″-deoxyavermectin A2a in 0.5 ml of methylene chloride is treated with 0.007 ml of acetic anhydride at room temperature for 1 hour. The reaction mixture is then diluted with ethylacetate and washed with dilute sodium bicarbonate solution and water, and is dried and concentrated in vacuo to a white foam, which is characterized by its mass spectrum and $^1$H-NMR spectrum as 4″-acetylamino-25-cyclopentyl-25-de-(1-methylpropyl)-4″-deoxyavermectin A2a.

## EXAMPLE 4

### 5-O-tert-Butyldimethylsilyl-25-cyclobutyl-25-de-(1-methylpropyl)avermectin B1a

A solution of 100 mg of 25-cyclobutyl-25-de-(1-methylpropyl)avermectin B1a, 48 mg of imidazole and 48 mg of tert-butyldimethylsilyl chloride in 1.0 ml of anhydrous dimethylformamide is stirred at room temperature for 50 minutes. Then the reaction mixture is poured into 5 ml of ice cold water and the aqueous phase is extracted four times with 25 ml of ether. The organic phase is washed twice with water, aqueous sodium chloride solution, dried with magnesium sulfate and concentrated in vacuo to a white foam. The crude product is purified by silica gel column chromatography with a methylene chloride : ethyl acetate-90:10 to 70:30 solvent system to give 5-O-tert-butyldimethylsilyl-25-cyclobutyl-25-de-(1-methylpropyl)avermectin B1a as an amorphous foam, which is characterized by its $^1$H-NMR- and mass spectra.

## EXAMPLE 5

### 5-O-tert-Butyldimethylsilyl-25-cyclobutyl-25-de-(1-methylpropyl)-4″-oxoavermectin B1a

To a solution containing 0.009 ml of oxalyl chloride in 0.5 ml of dry methylene chloride $CH_2Cl_2$ stirred at -60°C is added 0.015 ml of dry dimethylsulfoxide dissolved in 0.5 ml of dry methylene chloride during 15 min. Then a solution of 46.5 mg of 5-O-tert-butyldimethylsilyl-25-cyclobutyl-25-de-(1-methylpropyl)-

avermectin B1a dissolved in 0.5 ml of dry methylene chloride is added over a period of 15 minutes while maintaining the temperature at -60° C. The reaction mixture is stirred at this temperature for 30 minutes when 0.065 ml of dry triethylamine is added. The mixture is stirred for 5 additional minutes at -60° C, and then the cooling bath is removed and the reaction mixture is allowed to come to ambient temperature. After addition of water the reaction product is extraxted with methylene chloride, the extract is washed with water, dried and concentrated in vacuo to a yellow foam. This is identified by its mass and NMR spectra as 5-O-tert-butyldimethylsilyl-25-cyclobutyl-25-de-(1-methylpropyl)-4″-oxoavermectin B1a, which is used for further chemical reactions without purification.

## EXAMPLE 6

### 5-O-tert-Butyldimethylsilyl-25-cyclobutyl-25-de-(1-methylpropyl)-4″-deoxy-4″-methylaminoavermectin B1a

A solution of 2.6 ml of glacial acetic acid in 30 ml of methanol is treated with methylamine gas at 0° C until the pH of the solution reaches 9.0. To 0.5 ml of this solution 44.5 mg of 5-O-tert-butyldimethylsilyl-25-de-(1-methylpropyl)-25-cyclobutyl-4″-oxoavermectin B1a dissolved in 0.5 ml of methanol is added, and the reaction mixture is stirred at room temperature for 1 hour, when a solution of 3.5 mg of sodium cyanoborohydride in 0.25 ml of methanol is added dropwise over 10 min. After 50 min the reaction mixture is poured into 1.5 ml of cold aqueous sodium carbonate solution and the product is extracted with ether. The extract is washed with water, dried, and concentrated in vacuo to a yellow foam. Thin layer chromatography (silica gel, methylene chloride:ethyl acetate 85:15) of the crude product at this point shows several spots. Further purification by silica gel column chromatography using methylene chloride:ethyl acetate solvent mixtures gives 5-O-tert-butyldimethylsilyl-25-cyclobutyl-25-de-(1-methylpropyl)-4″-deoxy-4″-epimethylaminoavermectin B1a as the major product in form of a light foam, which is characterized by its mass and NMR spectra. Small amounts of 5-O-tert-butyldimethylsilyl-25-cyclobutyl-25-de-(1-methylpropyl)-4″-epiavermectin B1a and of 5-O-t-butyldimethylsilyl-25-cyclobutyl-25-de-(1-methylpropyl)-4″-deoxy-4″-methylaminoavermectin B1a are also obtained from this reaction mixture.

## EXAMPLE 7

### 25-Cyclobutyl-25-de-(1-methylpropyl)-4″-deoxy-4″-epimethylaminoavermectin B1a

A solution of 14 mg of 5-O-t-butyldimethylsilyl-25-cyclobutyl-25-de-(1-methylpropyl)-4″-deoxy-4″-epi-methylaminoavermectin B1a in 0.200 ml of methanol and a solution of 7 mg of p-toluenesulfonic acid monohydrate in 0.500 ml of methanol is mixed and stirred at room temperature for 45 minutes, and then poured into dilute aqueous sodium carbonate solution. The product is extracted with ethyl acetate, washed with water and dried over magnesium-sulfate, concentrated in vacuo, and purified by preparative silica gel thin layer chromatography with a methylene chloride:methanol 95:5 solvent mixture to give 25-cyclobutyl-25-de-(1-methylpropyl )-4″-deoxy-4″-epimethylaminoavermectin B1a, which is identified by NMR and mass spectra.

## EXAMPLE 8

### 25-Cyclobutyl-25-de-(1-methylpropyl)-4″-deoxy-22,23-dihydro-4″-methylaminoavermectin B1a

A solution of 100 mg of 25-cyclobutyl-25-de-(1-methylpropyl )-4″-deoxy-4″-methylaminoavermect in B1a and of 20 mg of tris(triphenylphosphine)rhodium(I) chloride in 6.0 ml of benzene is stirred at room temperature under an athmosphere of hydrogen for 18 hours. Then the reaction mixture is concentrated to dryness under a stream of $N_2$, and the residue is purified by preparative silica gel layer chromatography

with 9:1 $CH_2Cl_2$-MeOH as solvent. The major band is extracted and characterized as 25-cyclobutyl-25-de-(1-methylpropyl)-4″-deoxy-22,23-dihydro-4″-methylaminoavermectin B1a by its mass and NMR spectra.

## EXAMPLE 9

5-O-tert-Butyldimethylsilyl-25-de-(1-methylpropyl)-25-(1-methylpentyl)avermectin B2a

A solution of 100 mg of 25-de-(1-methylpropyl)-25-(1-methylpentyl) avermectin B2a, 48 mg of imidazole and 48 mg of tert-butyldimethylsilyl chloride in 1.0 ml of anhydrous dimethyl formamide is reacted in accordence with the procedure described in example 4 to give 5-O-tert-butyldimethylsilyl-25-de-(1-methylpropyl)-25-(1-methylpentyl)avermectin B2a, which is characterized by its mass and NMR spectra.

## EXAMPLE 10

5-O-tert-Butyldimethylsilyl-25-de-(1-methylpropyl)-25-(1-methylpentyl)-4″-oxoavermectin B2a

To a solution containing 0.0195 ml of oxalyl chloride in 1.2 ml of dry methylene chloride stirred at -78°C is added 0.0319 ml of dry dimethylsulfoxide dissolved in 0.6 ml of dry methylene chloride during 5 min. Then a solution of 210 mg of 5-O-tert-butyl dimethylsilyl-25-de-(1-methylpropyl)-25-(1-methylpentyl)-avermectin B2a dissolved in 1.5 ml of dry methylene chloride is added over a period of 10 minutes while maintaining the temperature at -78°C. The reaction mixture is stirred at this temperature for 45 minutes when 0.15 ml of dry triethylamine is added. The mixture is stirred for 5 additional minutes at -78°C, and then the cooling bath is removed and the reaction mixture is allowed to come to ambient temperature. After addition of water the reaction product is extracted with methylene chloride, the extract is washed with water, dried and concentrated in vacuo to a yellow foam. Silica gel thin layer chromatography shows this as a mixture of three products, which are isolated by preparative silica gel layer chromatography using a 75:25 methylene chloride:ethyl acetate solvent mixture. The products are identified by mass and NMR spectra as 5-O-tert-butyldimethylsilyl-25-de-(1-methylpropyl)-25-(1-methylpentyl)-4″,23-dioxoavermectin B2a, 5-O-tert-butyldimethylsilyl-25-de-(1-methylpropyl)-25-(1-methylpentyl)-4″-oxoavermectin B2a, and 5-O-tert-butyldimethylsilyl-25-de-(1-methylpropyl)-25-(1-methylpentyl)-23-oxoavermectin B2a.

## EXAMPLE 11

4″-Amino-5-O-tert-butyldimethylsilyl-25-de-(1-methylpropyl)-4″-deoxy-25-(1-methylpentyl)avermectin B2a

To a solution of 197 mg of 5-O-tert-butyldimethylsilyl-25-de-(1-methylpropyl)-25-(1-methylpentyl)-4″-oxoavermectin B2a and 179 mg of ammonium acetate in 2.55 ml of methanol stirred at room temperature is added a solution of 19 mg of sodium cyanoborohydride in 0.5 ml of MeOH. After 30 minutes the reaction mixture is added to 30 ml of dilute aqueous sodium bicarbonate solution, and the product is extracted with EtOAC. The extract is washed, dried with magnesium sulfate and concentrated in vacuo to a light glass. Purification via preparative silica gel layer chromatography gives the two 4″-epimers of 4″-amino-5-O-tert-butyl dimethylsilyl-25-de-(1-methylpropyl)-4″-deoxy-25-(1-methylpentyl)avermectin B2a, which are isolated by preparative silica gel layer chromatography and characterized by their mass and NMR spectra.

## EXAMPLE 12

4″-Acetylamino-5-O-tert-butyldimethylsilyl-25-de-(1-methylpropyl )-4″-deoxy-25-(1-methylpentyl)avermectin B2a

A solution of 56 mg of 4″-amino-5-O-tert-butyldimethylsilyl-5-de-(1-methylpropyl)-4″-deoxy-25-(1-methylpentyl)avermectin B2a in 0.5 ml of methylene chloride is treated with 0.007 ml of acetic anhydride according to the procedure fully described in example 3 to give 4″-acetylamino-5-O-tertbutyldimethylsilyl-25-de-(1-methylpropyl)-4″-deoxy-25-(1-methylpentyl)avermectin B2a, which is characterized by its mass and NMR spectra.

EXAMPLE 13

4″-Acetylamino-25-de-(1-methylpropyl)-4″-deoxy-25-(1-methylpentyl)avermectin B2a

A solution of 15 mg of 4″-acetylamino-5-O-t-butyldimethylsilyl-25-de-(1-methylpropyl)-4″-deoxy-25-(1-methylpentyl)avermectin B2a in 0.200 ml of methanol and a solution of 7 mg of p-toluenesulfonic acid monohydrate in 0.500 ml of methanol is reacted as fully described in example 7 to give 4″-acetylamino-25-de-(1-methylpropyl )-4″-deoxy-25-(1-methylpentyl)-avermectin B2a, which is identified by NMR and mass spectra.

EXAMPLE 14

4″-Acetylamino-5-O-tert-butyldimethylsilyl-25-de-(1-methylpropyl)-4″-deoxy-25-(1-methylpentyl)-23-O-(phenoxythionocarbonyl)avermectin B2a

In a test tube a solution of 50 mg of 4″-acetylamino-5-O-tert-butyldimethylsilyl-25-de-(1-methylpropyl)-4″-deoxy-25-(1-methylpentyl)avermectin B2a 15 drops of pyridine, and 3 drops of phenyl chlorothionocarbonate is stirred magnetically over-night at room temperature under a nitrogen atmosphere. Then water is added, and the solution is extracted with ether. The ether extract is washed with water repeatedly, then dried over magnesium sulfate and concentrated in vacuo to a thick oil. This is dissolved in methylene chloride and purified by preparative silica gel layer chromatography to give 4″-acetylamino-5-O-tert-butyldimethylsilyl-25-de-(1-methylpropyl)-4″-deoxy-25-(1-methylpentyl)-23-O-(phenoxythionocarbonyl)-avermectin B2a, which is characterized by its mass and NMR spectra.

EXAMPLE 15

4″-Acetylamino-5-O-tert-butyldimethylsilyl-25-de-(1-methylpropyl)-4″,23-dideoxy-25-(1-methylpentyl)-avermectin B2a

A solution containing 50 mg of 4″-acetylamino-5-O-tert-butyldimethylsilyl-25-de-(1-methylpropyl)-4″-deoxy-25-(1-methylpentyl)-23-O-(phenoxythionocarbonyl)avermectin B2a, 0.2 ml of tributyltin hydride, and 10 mg of 2,2′-azobis(2-methyl propionitrile) in 3.5 ml of toluene is refluxed under nitrogen for two hours. Then most of the solvent is evaporated under a stream of nitrogen, and the residue is taken up in methylene chloride. The solution is filtered through a column of 25 g of silica gel first with methylene chloride to wash off the tin compounds, then the product is eluted with EtOAc, and the EtOAc solution is concentrated in vacuo to a light oil. Purification by preparative silica gel layer chromatography gives 4″-acetylamino-5-O-tert-butyldimethylsilyl-25-de-(1-methylpropyl)-4″, 23-dideoxy-25-(1-methylpentyl)-avermectin B2a, which is characterized by its mass and NMR spectra.

EXAMPLE 16

4″-Acetylamino-25-de-(1-methylpropyl)-4″,23-dideoxy-25-(1-methylpentyl)avermectin B2a

A solution of 33 mg of 4″-acetylamino-5-O-tert-butyldimethylsilyl-25-de-(1-methylpropyl )-4″,23-dideoxy-25-(1-methylpentyl)avermectin B2a in 2.0 ml of an anhydrous hydrogen fluoride - pyridine -tetrahydrofuran mixture, prepared by mixing 14.0 ml of tetrahydrofuran, 4.0 ml of pyridine and 2.0 ml of commercial HF - pyridine (consisting of 70 % hydrogen fluoride and 30 % of pyridine, supplied by Aldrich Chemical Company) is kept at room temperature for two days. Then the reaction mixture is poured into dilute aqueous sodium bicarbonate solution and extracted with ethyl acetate to give 4″-acetylamino-25-de-(1-methylpropyl)-4″,23-dideoxy-25-(1-methylpentyl)avermectin B2a as a glass, which is characterized by its UV, mass, and NMR spectra.

EXAMPLE 17

5-O-tert-Butyldimethylsilyl-25-de-(1-methylpropyl)-25-(1-methylpentyl)-4″-oxoavermectin B2a semicarbazone

To a solution of 120 mg of 5-O-tert-butyldimethylsilyl-25-de-(1-methylpropyl)-25-(1-methylpentyl)-4″-oxoavermectin B2a in 1.2 ml of methanol is added 120 mg of semicarbazide hydrochloride followed by 0.6 ml of pyridine. The reaction mixture is stirred for 24 hours at room temperature, and is then concentrated in vacuo to a solid residue. This is taken up with 8 ml of 5% aqueous ammonium chloride solution and extracted with methylene chloride. The methylene chloride extract is concentrated to an oil and then purified by preparative silica gel layer chromatography to give 5-O-tert-butyl-dimethylsilyl-25-de-(1-methylpropyl)-25-(1-methylpentyl)-4″-oxoavermectin B2a semicarbazone, which is characterized by its mass and NMR spectra.

EXAMPLE 17a

When 5-O-tert-butyldimethylsilyl-25-de-(1-methylpropyl)-25-(1-methylpentyl)-4″-oxoavermectin B2a is reacted with 4-methylsemicarbazide hydrochloride or 4,4-dimethylsemicarbazide hydrochloride instead of semicarbazide hydrochloride according to the procedure fully described in example 17, the corresponding 5-O-tert-butyldimethylsilyl-25-de-(1-methylpropyl)-25-(1-methylpentyl)-4″-oxoavermectin B2a (4-methylsemicarbazone) and 5-O-tert-butyldimethylsilyl-25-de-(1-methylpropyl)-25-(1-methylpentyl)-4″-oxoavermectin B2a (4,4-dimethylsemicarbazone) are obtained.

EXAMPLE 18

25-De-(1-methylpropyl)-25-(1-methylpentyl)-4″-oxoavermectin B2a semicarbazone.

55 Mg of 5-O-tert-butyl-dimethyl silyl-25-de-(1-methylpropyl)-25-(1-methylpentyl)-4″-oxoavermectin B2a semicarbazone is deprotected with 0.6 ml of hydrogen fluoride-pyridine-tetrahydrofuran solution as fully described in example 16 to give 25-de-(1-methylpropyl)-25-(1-methylpentyl)-4″-oxoavermectin B2a semicarbazone, which is characterized by its mass and NMR spectra.

EXAMPLE 18a.

When 5-O-tert-butyldimethylsilyl-25-de-(1-methylpropyl)-25-(1-methylpentyl)-4″-oxoavermectin B2a (4-methylsemicarbazone) or 5-O-tert-butyldimethylsilyl-25-de-(1-methylpropyl)-25-(1-methylpentyl)-4″-oxoavermectin B2a (4,4-dimethylsemicarbazone) are deprotected as fully described in EXAMPLE 16 one obtains the corresponding 25-de-(1-methylpropyl)-25-(1-methylpentyl)-4″-oxoavermectin B2a (4-methylsemicarbazone) or 25-de-(1-methylpropyl)-25-(1-methylpentyl)-4″-oxoavermectin B2a (4,4-dimethylsemicarbazone) derivatives.

## EXAMPLE 19

### 25-De-(1-methylpropyl)-25-(1-methylpentyl)avermectin B2a 4″-ketoxime.

A solution of 100 mg of 5-O-tert-butyldimethylsilyl-25-de-(1-methylpropyl)-25-(1-methylpentyl)-4″-oxoavermectin B2a and 0.1 ml of O-(trimethylsilyl)-hydroxylamine in 2.0 ml of tetrahydrofuran is kept at room temperature for 3 days. The reaction mixture is concentrated in vacuo to an oily residue. This is without purification deprotected according to the procedure fully described in EXAMPLE 16. Purification by preparative silica gel layer chromatography gives 25-de-(1-methylpropyl)-25-(1-methylpentyl)avermectin B2a 4″-ketoxime, which is characterized by its mass and NMR spectra.

## EXAMPLE 20

### 25-De-(1-methylpropyl)-25-(1-methylpentyl)-4″-oxoavermectin B2a acetylhydrazone.

A solution of 200 mg of 5-O-tert-butyl dimethylsilyl-25-de-(1-methylpropyl)-25-(1-methylpentyl)-4″-oxoavermectin B2a, 35 mg of acetylhydrazide, 25 ml of glacial acetic acid and 100 ml of pyridine in 1.2 mL of methanol is stirred at room temperature for 18 hours. Then methylene chloride is added to the reaction mixture, the solution is washed with aqueous sodium bicarbonate solution and water, dried, and concentrated in vacuo to a light colored glass. This is used without further purification for the deprotection step fully described in example 16. Purification of the crude product by preparative silica gel layer chromatography gives 25-de-(1-methylpropyl)-25-(1-methylpentyl)-4″-oxoavermectin B2a acethydrazone which is characterized by its mass and NMR spectra.

## EXAMPLE 21

### 4″-O-Pivaloyl-25-de-(1-methylpropyl)-25-(1-methylpentyl)-avermectin B2a

A solution of 100 mg of 5-O-tert-butyl dimethylsilyl-25-de-(1-methylpropyl)-25-(1-methylpentyl)-avermectin B2a, 50 mg of 4-dimethylaminopyridine and 50 mg of diisopropylethylamine in 2.5 ml of methylene chloride is stirred in an ice bath while adding a solution of 36 mg of pivaloyl chloride in 0.5 ml of methylene chloride. After 1 hour at ice bath temperature the reaction mixture is diluted with methylene chloride, and the solution is washed with aqueous sodium bicarbonate solution and water, dried, and concentrated in vacuo to a light colored glass. This is used without further purification for the deprotection step fully described in example 16. Purification of the crude product by preparative silica gel layer chromatography gives 4″-O-pivaloyl-25-de-(1-methylpropyl)-25-(1-methylpentyl)-avermectin B2a, which is characterized by its mass and NMR spectra.

## EXAMPLE 22

5-O-tert-butyldimethylsilyl-25-de-(1-methylpropyl)-25-(1-methylpentyl)-4″-O-(4-nitrophenoxycarbonyl)-avermectin B2a

To a solution of 220 mg of 5-O-tert-butyldimethylsilyl-25-de-(1-methylpropyl)-25-(1-methylpentyl)-avermectin B2a, 107 mg of 4-dimethyL aminopyridine, and 0.15 ml of diisopropylethylamine in 5.5 ml of methylene chloride stirred at 0 °C is added a solution of 130 mg of p-nitrophenylchloroformate in 2.2 ml of methylene chloride. After 20 minutes at 0°C the reaction mixture is diluted with 40 ml of ether and washed consecutively with 5% aqueous potassium phosphate, monobasic solution, water, and saturated sodium chloride solution, dried over magnesium sulfate, and concentrated in vacuo to a light foam. Purification by preparative silica gel layer chromatography gives 5-O-tert-butyldimethylsilyl-25-de-(1-methylpropyl)-25-(1-methylpentyl)-4″-O-(4-nitrophenoxycarbonyl)avermectin B2a, which is characterized by its mass and NMR spectra.

EXAMPLE 23

4″-Aminocarbonyloxy-5-O-tert-butyldimethylsilyl-25-de-(1-methylpropyl)-25-(1-methylpentyl)avermectin B2a

A solution of 120 mg of 5-O-tert-butyl dimethylsilyl-25-de-(1-methylpropyl)-25-(1-methylpentyl)-4″-O-(4-nitrophenoxycarbonyl)avermectin B2a in 7 ml of ether is treated with a stream of gaseous ammonia and left 18 hours at room temperature. Then the reaction mixture is concentrated in vacuo at room temperature, dissolved in ether, washed with water and saturated sodium chloride solution, dried over magnesium-sulfate, and concentrated to a light foam. Purification by preparative silica gel layer chromatography with 1 to 10 % ethyl acetate in methylene chloride as solvent gives 4″-aminocarbony loxy-5-O-tert-butyl-dimethylsilyl-25-de-(1-methylpropyl)-25-(1-methylpentyl)avermectin B2a, which is characterized by its mass and NMR spectra.

EXAMPLE 24

4″-Aminocarbonyloxy-25-de-(1-methylpropyl)-25-(1-methylpentyl)avermectin B2a

When 4″-aminocarbonyloxy-5-O-tert-butyldimethylsilyl-25-de-(1-methylpropyl)-25-(1-methylpentyl)-avermectin B2a is deprotected as described in EXAMPLE 16 one obtains 4″-aminocarbonyloxy-25-de-(1-methylpropyl)-25-(1-methylpentyl)avermectin B2a, which is characterized by its mass and NMR spectra.

EXAMPLE 25

5-O-tert-Butyldimethylsilyl-4″-O-methoxymethyl-25-de-(1-methylpropyl)-25-(1-methylpentyl)avermectin B2a

A solution of 295 mg of 5-O-tert-butyldimethylsilyl-25-de-(1-methylpropyl)-25-(1-methylpentyl)-avermectin B2a, 0.16 ml of diisopropylethylamine and 0.025 ml of chloromethyl methyl ether in 1.0 ml of methylene chloride is kept at room temperature for 20 hours. Then the solution is diluted with methylene chloride, washed with aqueous sodium bicarbonate, water and saturated sodium chloride solution, dried over magnesium sulfate, and concentrated in vacuo to a light foam. Purification by preparative silica gel layer chromatography gives 5-O-tert-butyl-dimethylsilyl-4″-O-methoxymethyl-25-de-(1-methylpropyl)-25-(1-methylpentyl)avermectin B2a, which is characterized by its mass and NMR spectra.

EXAMPLE 26

4″-O-Methoxymethyl-25-de-(1-methylpropyl)-25-(1-methylpentyl)avermectin B2a

5-O-tert-butyldimethylsilyl-4″-O-methoxymethyl-25-de-(1-methylpropyl )-25-(1-methylpentyl)avermectin B2a is deprotected with a hydrogen fluoride - pyridine - tetrahydrofuran mixture according to the procedure of example 16 to give 4″-O-methoxymethyl-25-de-(1-methylpropyl)-25-(1-methylpentyl)avermectin B2a, which is characterized by its mass and NMR spectra.

## EXAMPLE 27

25-De-(1-methylpropyl)-25-(1-methylpentyl)avermectin B2a monosaccharide.

A solution containing 500 mg of 25-de-(1-methylpropyl)-25-(1-methylpentyl)avermectin B2a and 0.1 ml of sulfuric acid in 9.9 ml of 2-propanol is kept at room temperature for 16 hours. Then 125 ml of chloroform is added and the solution is washed with aqueous saturated sodium bicarbonate solution and water, dried, and concentrated in vacuo to a yellow foam. Purification by preparative silica gel layer chromatography with a benzene-ethyl acetate solvent mixture gives 25-de-(1-methylpropyl)-25-(1-methyl pentyl)-avermectin B2a monosaccharide, which is characterized by its mass and NMR spectra.

## EXAMPLE 28

5-O-tert-Butyldimethylsilyl-25-de-(1-methylpropyl)-25-(1-methylpentyl)avermectin B2a monosaccharide.

Reaction of 100 mg of 25-de-(1-methylpropyl)-25-(1-methylpentyl)avermectin B2a monosaccharide with 50 mg of imidazole and 50 mg of tert-butyl-dimethylsilyl chloride in 1.0 ml of anhydrous dimethylformamide according to the procedure of example 4 gives 5-O-tert-butyl-dimethylsilyl-25-de-(1-methylpropyl)-25-(1-methyl-pentyl)avermectin B2a monosaccharide.

## EXAMPLE 29

5-O-tert-Butyldimethylsilyl-25-de-(1-methylpropyl)-25-(1-methylpentyl)-4″-oxoavermectin B2a monosaccharide

Reaction of 45 mg of 5-O-tert-butyl dimethylsilyl-25-de-(1-methylpropyl)-25-(1-methylpentyl)avermectin B2a monosaccharide with 0.009 ml of oxalyl chloride and 0.015 ml of dimethylsulfoxide in methylene chloride followed by 0.065 ml triethylamine according to the procedure of example 5 gives 5-O-tert-butyldimethylsilyl-25-de-(1-methylpropyl)-25-(1-methylpentyl)-4″-oxoavermectin B2a monosaccharide.

## EXAMPLE 30

4″-Amino-5-O-tert-butyldimethylsilyl-25-de-(1-methylpropyl)-25-(1-methylpentyl)-4″-deoxyavermectin B2a monosaccharide.

Reaction of 170 mg of 5-O-tert-butyldimethylsilyl-25-de-(1-methylpropyl)-25-(1-methylpentyl)-4″-oxoavermectin B2a monosaccharide with 180 mg of ammonium acetate and 20 mg of sodium cyanoborohydride in 3.0 ml of methanol according to the procedure of example 11 gives the two 4″-epimers of 4″-amino-5-O-tert-butyldimethylsilyl-25-de-(1-methyl-propyl)-25-(1-methylpentyl)-4″-deoxyavermectin B2a

monosaccharide, which are isolated by preparative silica gel layer chromatography and characterized by their mass and NMR spectra.

## EXAMPLE 31

5-O-tert-Butyldimethylsilyl-4″-deoxy-4″-N,N-dimethylamino-25-de-(1-methylpropyl)-25-(1-methylpentyl)-avermectin B2a monosaccharide.

A solution containing 45 mg of 4″-amino-5-O-tert-butyldimethylsilyl-4″-deoxy-25-de-(1-methylpropyl)-25-(1-methylpentyl)avermectin B2a monosaccharide, 0.4 ml of glacial acetic acid, 0.5 ml of 37% aqueous formaldehyde solution in 0.7 ml of methanol is stirred for 30 minutes at room temperature, then cooled in an ice bath, and 130 mg of sodium borohydride is added in 10 mg portions every 5 to 10 minutes. Then the reaction mixture is neutralised with a saturated, aqueous sodium bicarbonate solution, and the product is extracted with ether. The ether extract is washed with dilute aqueous sodium bicarbonate solution and water, dried and concentrated in vacuo to a glass. Purification by preparative silica gel layer chromatography with ethyl acetate gives 5-O-tert-butyldimethylsilyl-4″-deoxy-4″-N,N-dimethylamino-25-de-(1-methylpropyl)-25-(1-methylpentyl)avermectin B2a monosaccharide, which is characterized by its mass and NMR spectra.

## EXAMPLE 32

4″-Deoxy-4″-N,N-dimethylamino-25-de-(1-methylpropyl)-25-(1-methylpentyl)avermectin B2a monosaccharide.

5-O-tert-Butyldimethylsilyl-4″-deoxy-4″-N,N-dimethylamino-25-de-(1-methylpropyl)-25-(1-methylpentyl)-avermectin B2a monosaccharide is deprotected with a hydrogen fluoride - pyridine - tetrahydrofuran mixture according to the procedure of example 16 to give 4″-deoxy-4″-N,N-dimethylamino-25-de-(1-methylpropyl)-25-(1-methylpentyl)avermectin B2a monosaccharide, which is characterized by its mass and NMR spectra.

## EXAMPLE 33

5-O-tert-Butyldimethylsilyl-4″-deoxy-4″-methoxycarbonylamino-25-de-(1-methylpropyl)-25-(1-methylpentyl)-avermectin B2a

A solution of 206 mg of 4″-amino-5-O-tert-butyl-dimethylsilyl-4″-deoxy-25-de-(1-methylpropyl)-25-(1-methylpentyl)avermectin B2a and 0.14 ml of diisopropylethylamine in 2.5 ml of methylene chloride is stirred in an ice bath, while 17 microliter of methyl chloroformate is added. After 15 minutes the ice bath is removed and the reaction mixture is left at room temperature for 24 hours. Then more methylene chloride is added, and the solution is washed with water and saturated sodium chloride solution, dried over magnesium sulfate, and concentrated in vacuo to a light foam. Purification by preparative silica gel layer chromatography gives 5-O-tert-butyldimethylsilyl-4″-deoxy-4″-methoxycarbonylamino-25-de-(1-methylpropyl)-25-(1-methylpentyl)avermectin B2a, which is characterized by its mass and NMR spectra.

## EXAMPLE 34

4″-Deoxy-4″-methoxycarbonylamino-25-de-(1-methylpropyl)-25-(1-methylpentyl)avermectin B2a

A solution of 150 mg of 5-O-tert-butyldimethylsilyl-4″-deoxy-4″-methoxycarbonylamino-25-de-(1-methylpropyl)-25-(1-methylpentyl)avermectin B2a in 2.0 ml of methanol is reacted with a solution of 70 mg of p-toluenesulfonic acid monohydrate in 5.0 ml of methanol at room temperature for 30 minutes as described in example 7 to give 4″-deoxy-4″-methoxycarbonylamino-25-de-(1-methylpropyl)-25-(1-methylpentyl)-avermectin B2a, which is characterized by its mass and NMR spectra.

## Claims

1. A compound having the formula:

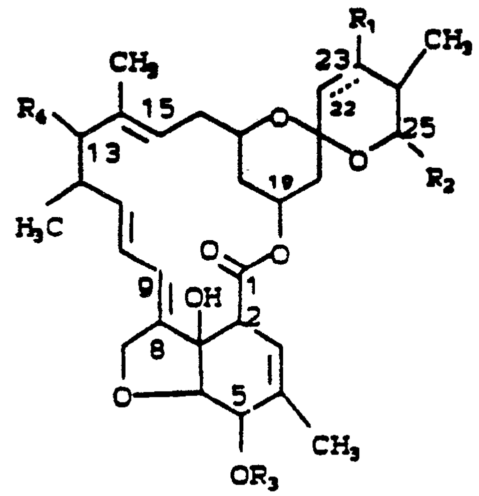

wherein the broken line at the 22,23-position represents a single bond and wherein $R_1$ is hydrogen or hydroxy, or the broken line represents a double bond and $R_1$ is absent;
$R_2$ is an alpha-branched $C_3$-$C_8$ alkyl, alkenyl, alkoxyalkyl or alkylthioalkyl group; a $C_5$-$C_8$ cycloalkyl substituted $C_1$-$C_4$ group; a $C_3$-$C_8$ cycloalkyl or $C_5$-$C_8$ cycloalkenyl group, either of which may optionally be substituted by methylene or one or more $C_1$-$C_4$ alkyl groups or halo atoms; or a 3 to 6 membered oxygen or sulfur containing heterocyclic ring which may be saturated, or fully or partly unsaturated and which may optionally be substituted by one or more $C_1$-$C_4$ alkyl groups or halo atoms, provided $R_2$ is not 2-propyl,2-butyl, 2-buten-2-yl, 2-penten-2-yl or 4-methyl-2-penten-2-yl;
$R_3$ is hydrogen, loweralkyl, or loweralkanoyl;
$R_4$ is

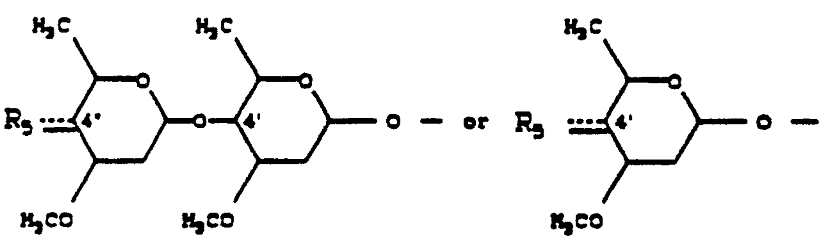

where the broken line at the 4′ and 4″ positions indicates a double bond and $R_5$ is ketone, oxime, hydrazone, loweralkanoylhydrazone, semicarbazone, N-loweralkyl semicarbazone or N,N-diloweralkyl semicarbazone; or the broken line at the 4′ and 4″ positions indicates a single bond and $R_5$ is amino, loweralkylamino, diloweralkylamino, lower alkanoylamino, loweralkoxycarbonylamino, carbamoyloxy, N-loweralkylcarbamoyloxy, N,N-diloweralkylcarbamoyloxy, loweralkoxy, loweralkanoyloxy, or loweralkoxycarbonyloxy.

2. A compound of Claim 1 wherein the broken line at the 22,23-position represents a single bond and wherein $R_1$ is hydrogen or hydroxy, or the broken line represents a double bond and $R_1$ is absent;
$R_2$ is an alpha branched $C_3$-$C_8$ alkyl or alkenyl group; a $C_5$-$C_8$ cycloalkyl substituted $C_1$-$C_4$ alkyl group; a

$C_3$-$C_8$ cycloalkyl or $C_5$-$C_8$ cycloalkenyl group, either of which may optionally be substituted by methylene or one or more $C_1$-$C_4$ alkyl groups or halo atoms;
$R_3$ is hydrogen;
$R_4$ is

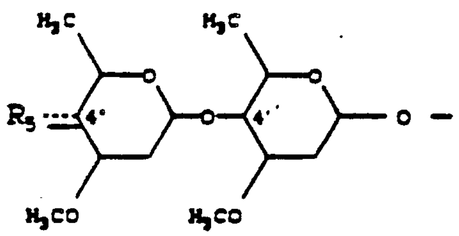
or
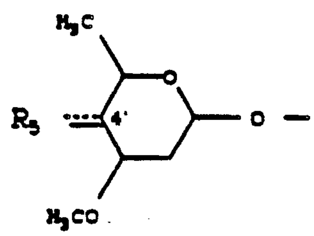

where the broken line at the $4'$ and $4''$ positions indicates that $R_5$ is connected by a double bond and $R_5$ is ketone, loweralkanoylhydrazone, semicarbazone, N-loweralkylsemicarbazone or N,N- diloweralkylsemicarbazone, or the broken line at the $4'$ and $4''$ positions indicates that $R_5$ is connected by a single bond and $R_5$ is amino, loweralkylamino, diloweralkylamino, lower alkanoylamino, loweralkyloxycarbonylamino, carbamoyloxy, N-loweralkylcarbamoyloxy, or N,N-diloweralkylcarbamoyloxy.

3. A compound of Claim 1 wherein the broken line at the 22,23-position represents a single bond and wherein $R_1$ is hydrogen or hydroxy, or the broken line represents a double bond and $R_1$ is absent;
$R_2$ is 2-pentyl, 2-hexyl cyclobutyl, cyclopentyl, cyclohexyl, 3-thiophenyl, 1-methylthioethyl;
$R_3$ is hydrogen;
$R_4$ is

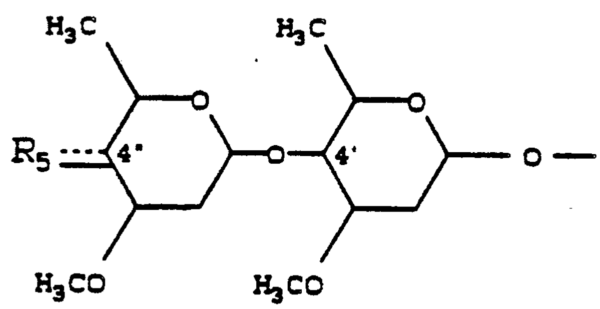

where the broken line at the $4''$ position indicates that $R_5$ is connected by double bond and $R_5$ is ketone, loweralkanoylhydrazone, semicarbazone, N-loweralkylsemicarbazone or N,N- diloweralkylsemicarbazone, or the broken line at the $4''$ position indicates that $R_5$ is connected by a single bond and $R_5$ is amino, loweralkylamino, diloweralkylamino, lower alkanoylamino, loweralkyloxycarbonylamino.

4. 25-Cyclobutyl-25-de-(1-methylpropyl)-$4''$-deoxy-$4''$-epi-methylaminoavermectin Bla.

5. 25-cyclobutyl-25-de-(1-methylpropyl)-$4''$-deoxy-22,23-dihydro-$4''$-methylaminoavermectin Bla.

6. A process for the preparation of the compounds of Claim 1 with an acyl function at the 5, $4'$ or $4''$, positions which comprises acylating the starting material with a hydroxy group at the 5, 4 or $4''$, positions.

7. A process for the preparation of the compounds of Claim 1 wherein the 22,23-position is a single bond and $R_1$ is hydrogen which comprises selectively reducing the 22,23-double bond of the corresponding starting material with Wilkinsons homogenous catalyst, triphenylphosphine rhodium chloride.

8. A process for the preparation of the compound of Claim 1 wherein $R_5$ is a ketone or an amino function which comprises selectively oxidizing the $4'$ or $4''$ hydroxy group to a ketone followed by amination of the ketone function.

9. The use of a compound as claimed in Claim 1 for the preparation of a medicament useful for the treatment of parasitic infections.

10. A composition for the treatment of parasitic infections in animals which comprises an inert carrier and a compound of Claim 1.

European Patent Office

# EUROPEAN SEARCH REPORT

Application number

EP 89313349.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A1 - 0 284 255 (MERCK & CO. INC.) * Claims 1,11,13 * | 1-3,6-10 | C 07 H 17/08<br>C 07 D 493/22<br>A 61 K 31/70<br>A 01 N 43/90 |
| A | EP - A2 - 0 276 131 (PFIZER INC.) * Claims 1,11 * | 1-3,9,10 | |
| D,A | EP - A2/A3 - 0 214 731 (PFIZER) * Claims 1,13 * | 1-3,9,10 | |
| A | EP - A1 - 0 266 131 (MERCK & CO. INC.) * Claims 1,12; pages 7,8 * | 1-3,6-10 | |
| A | EP - A1 - 0 089 202 (MERCK & CO.INC.) * Claims 1-5,8-10; pages 14-17 * | 1-3,7-10 | |

TECHNICAL FIELDS SEARCHED (Int Cl.5)

C 07 H 17/00
C 07 D 493/00
C 07 H 19/00

The present search report has been drawn up for all claims

| Place of search | Date of completion of the search | Examiner |
|---|---|---|
| VIENNA | 22-03-1990 | PETROUSEK |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document